# EUROPÄISCHE PATENTANMELDUNG

(11) **EP 1 541 136 A1**
(43) Veröffentlichungstag der Anmeldung: **15.06.2005**
(21) Anmeldenummer: 03028353.5
(22) Anmeldetag: 10.12.2003
(51) Int. Cl.: A61K 9/70, A61K 9/06, A61K 47/38, A61K 47/32, A61K 31/565, A61K 7/42

(54) **UV-Licht stabile halbfeste transdermale Systeme, die einen lichtempfindlichen Wirkstoff und einen UV-Absorber enthalten**

(71) Anmelder: Schering AG, 13342 Berlin (DE)
(72) Erfinder: Podhaisky, Hans-Peter, 07745 Jena (DE); Bracht, Stefan, 07751 Jena (DE)
(74) Vertreter: Cramer, Eva-Maria

(57) **Zusammenfassung**

Die Erfindung betrifft ein neues halbfestes transdermales therapeutisches System (halbfestes TTS), vorzugsweise ein Gel, welches einen lichtempfindlichen pharmazeutischen Wirkstoff und mindestens einen UV-Absorber ohne pharmakologische Wirksamkeit in den eingesetzten Konzentration enthält und wobei dieser UV-Absorber im halbfesten TTS gelöst oder dispergiert vorliegt.
Das erfindungsgemäße halbfeste TTS realisiert eine hohe Stabilität ohne die Nachteile herkömmlicher lichtempfindlicher Wirkstoffe enthaltender (1) halbfester (2) TTS.

## Beschreibung

Die Erfindung betrifft ein halbfestes transdermales therapeutisches System (halbfestes TTS) mit einem lichtempfindlichen pharmazeutischen Wirkstoff und mindestens einem UV-Absorber ohne pharmakologische Wirksamkeit in den eingesetzten Konzentrationen, wobei dieser im halbfesten transdermalen System, vorzugsweise ein Gel, gelöst oder dispergiert vorliegt.
Es ist aus der Patent- und Fachliteratur bekannt, dass eine transdermale Applikation von pharmazeutisch wirksamen Substanzen verschiedene Vorteile gegenüber einer oralen Zufuhr des Arzneistoffes aufweist, wie beispielsweise
- keine first pass Metabolisierung des Wirkstoffes durch Umgehung des Gastrointestinaltraktes,
- gleichmäßige Freisetzung des Wirkstoffes über mehrere Stunden, bedingt durch die Depotfunktion der Haut
- dadurch die Aufrechterhaltung eines gleichbleibenden Plasmaspiegels.

Bei den bekannten transdermalen therapeutischen Systemen haben sich bisher im Wesentlichen zwei verschiedene Arzneiformen durchsetzen können. Es existiert eine große Anzahl von therapeutischen Pflastern, die den Wirkstoff in einer Matrix gelöst oder suspendiert enthalten.
In den letzten Jahren haben sich darüber hinaus halbfeste transdermale therapeutische Systeme und hier vor allem Gele, etabliert. Ein wesentlicher Vorteil dieser Form gegenüber den transdermalen Pflastern besteht in der besseren Hautverträglichkeit der nicht-okklusiven Arzneiform. Weitere Vorzüge werden in einer individuellen Dosierbarkeit und darin gesehen, dass der Anwender nicht sichtbar als kranke oder anderweitig behandlungswürdige Person stigmatisiert wird.
Verschiedene pharmazeutische Wirkstoffe, die sich zur transdermalen Applikation eignen (z.B. Nifedipin, Nicotin, Arylpropionsäure- und Benzophenonderivate, Gestoden, Levonorgestrel, Estradiol und andere zur transdermalen Applikation geeigneten Hormone), sind lichtempfindlich. Lichtempfindliche Substanzen absorbieren Strahlung innerhalb des Wellenlängenspektrums des Sonnenlichtes, vor allem innerhalb des ultravioletten Bereiches (UV, 280-400 nm). Durch Lichtexposition von pharmazeutischen Zubereitungen mit lichtempfindlichen Wirkstoffen kann es zu einem photochemischen Abbau der Wirkstoffe und damit im Extremfall zu einem Wirkverlust der Zubereitung kommen. Darüber hinaus können die Abbauprodukte Ursache für phototoxische oder photoallergische Reaktionen der Haut sein. Beispielsweise ist bekannt, daß Ketoprofen-Gele wie auch andere Arylpropionsäure- und Benzophenonderivate unter dem Einfluß von Sonnenlicht zu einem gehäuften Auftreten von phototoxischen und teilweisen photoallergischen Hautreaktionen führen, die auf photosensibilisierende Eigenschaften dieser Substanzklasse zurückzuführen sind. Um derartige Zersetzungsvorgänge zu verhindern, müssen für transdermale Zubereitungen mit lichtempfindlichen Wirkstoffen geeignete Schutzmaßnahmen getroffen werden.
Bei den bisher bekannten transdermalen Gelen findet der Lichtschutz bisher nur insoweit Beachtung, dass die Zubereitungen in lichtundurchlässigen Verpackungen, meist aluminisierte Siegelrandbeutel / Sachets, konfektioniert sind. Diese Methode des Lichtschutzes von halbfesten TTS während der Lagerung allein kann aber unzureichend sein. Sie setzt voraus, dass der lichtempfindliche Wirkstoff nach dem Auftragen der Zubereitung auf die Haut nicht mehr dem Sonnenlicht ausgesetzt ist. Der Schutz vor Sonnenlicht kann z.B. durch das Tragen von Kleidung über der Applikationsstelle erfolgen. Dies stellt jedoch für den Anwender insbesondere in klimatisch warmen Gegenden oder während klimatisch warmer Perioden des Jahres im Zusammenhang mit geringer Bekleidung eine unerwünschte Beschränkung der Anwendungsorte am Körper dar.

Es ist bekannt, dass der UV-Anteil des Sonnenlichtes in die Haut eindringt. So durchdringt UVB-Licht (280-320) die gesamte Epidermis bis zum Stratum basale. Das längerwellige UVA-Licht (320-400 nm) dringt bis in das Bindegewebe vor. Transdermale Gele werden in der Regel sehr großflächig angewandt (100-200 cm² und mehr), und der vollständige Wirkstofftransport von der äußersten Schicht der Haut (Stratum corneum) bis in die systemische Zirkulation nimmt bis zu 24 Stunden in Anspruch. Vor allem das Stratum corneum als Hauptpenetrationsbarriere fungiert als ein Wirkstoffreservoir, das große Teile des Arzneistoffes für mehrere Stunden speichert. In diesen Bereich kann das komplette Spektrum des UV-Lichtes vordringen und so photochemische Zersetzungsreaktionen verursachen.
Unter diesen Gesichtspunkten stellt sich die Problematik der Lichtempfindlichkeit während der Anwendung bei transdermalen Gelen noch stärker dar als bei transdermalen Pflastern. Bei den letzteren ist die exponierte Applikationsfläche in der Regel viel kleiner sowie weiterhin mindestens eine außen liegende Rückschicht aus einer Kunststofffolie vorgesehen, die in der Regel immer einen minimalen UV-Schutz bietet.

Aus der Patentliteratur sind halbfeste TTS (Gele) mit pharmazeutischen, auch lichtempfindlichen Wirkstoffen bekannt.
Die WO-A1-03/082960 beschreibt die Hestellung eines Gels, welches pharmazeutische Wirkstoffe enthalten kann. In der WO-A1-01/60399 wird ein Diclofenac enthaltendes Gel aufgezeigt und die Patentschrift WO-A2-02/051421 beschreibt eine Gelzusammensetzung mit mindestens einem androgenen Steroid zur Behandlung und/oder Prophylaxe von Hypogonadismus. Alle Patentschriften nehmen keinen Bezug auf die sensible Problematik der Lichtempfindlichkeit und des Lichtschutzes bei halbfesten TTS.

Aufgabe der Erfindung ist es deshalb, ein halbfestes transdermales therapeutisches System (halbfestes TTS) mit einem lichtempfindlichen pharmazeutischen Wirkstoff bereitzustellen, wobei das Applikationssystem eine hohe Stabilität ohne die bei Anwendung der konventionellen halbfesten transdermalen Applikationsformen bekannten Nachteile realisiert.
Dabei sollten die aus dem beabsichtigten Lichtschutz für den Anwender möglicherweise resultierende schädliche Begleitwirkungen, wie z.B. Aufnahme von Lichtschutzstoffen in den Körper, so gering wie möglich gehalten werden.

Erfindungsgemäß wird die Aufgabe durch ein halbfestes TTS mit einem lichtempfindlichen pharmazeutischen Wirkstoff und mindestens einem UV-Absorber ohne pharmakologische Wirksamkeit in den eingesetzten Konzentrationen gelöst, wobei dieser im halbfesten TTS gelöst oder dispergiert vorliegt.

Erfindungsgemäß kann das halbfeste TTS ein Gel sein. Auch Applikationsformen, wie Cremes, Lösungen und Suspensionen erweisen sich als vorteilhaft.

Die physiko-chemischen Kenngrößen,wie der Logarithmus der Verteilungskoeffizienten zwischen 1-Octanol und Wasser (Log P _{Oct/H2O} im folgenden LogP bezeichnet) sowie das Molekulargewicht, nehmen bei der Beurteilung des halbfesten TTS einen besonderen Stellenwert ein.

Ferner kann/können erfindungsgemäß bei dem halbfesten TTS der/die Verteilungskoeffizient/en [log P] des/der UV-Absorber/s größer als 2,0 oder kleiner als 1,0, vorzugsweise größer als 3,0 oder kleiner als 0,0 sein.

Auch kann beim erfindungsgemäßen halbfesten TTS die molare Masse des/der UV-Absorber/s größer als 150 gmol⁻¹ , vorzugsweise größer als 250 gmol⁻¹ sein.
Besonders bevorzugt ist/sind UV-Absorber mit einer molaren Masse größer 400 g/mol.

Bei dem erfindungsgemäßen halbfesten TTS kann /können der/die/UV-Absorber in einem Anteil von 1-10% enthalten sein , vorzugsweise 2-5%.
Sofern das erfindungsgemäße halbfeste TTS leichtflüchtige Bestandteile wie beispielsweise Ethanol, Methanol, Propanol oder DMSO enthält, bezieht sich der Mengenanteil auf die Summe der nicht-leichtflüchtigen Vehikelbestandteile oder anders ausgedrückt auf die nach dem Abgang aller flüchtigen Bestandteile auf der Haut verbleibende Formulierung.

Es konnten UV-absorbierenden Substanz(en) ermittelt werden, deren Absorptionsmaximum innerhalb des Wellenbereiches liegt, der für die photochemische Zersetzung des zu schützenden Wirkstoffes verantwortlich ist. Sofern ein Schutz über einen breiteren UV-Spektralbereich bzw. darinliegende Absorptinsmaxima des Wirkstoffes notwendig ist, erweist es sich als vorteilhaft, zwei UV-absorbierende Substanzen mit unterschiedlichen Absorptionsmaxima zu kombinieren.

Weiterhin kann/können bei dem erfindungsgemäßen halbfesten TTS die im UV-Bereich absorbierende/n Substanz/en aus der Gruppe ausgewählt sein, die p-Aminobenzoesäure und Aminobenzoesäurederivate, vorzugsweise 4-Dimethylaminobenzoesäure-2-ethyl-hexylester, sowie Zimtsäure und ihre Derivate, vorzugsweise 4-Methoxyzimtsäureisoamyleste sowie 3-Benzylidenbornan-2-on und Benzylidenbornan- 2-on-Derivate, vorzugsweise 3-(4')Methylbenzyliden-bornan- 2-on, 3-(4-Sulfo)benzylidenbornan-2-on"sowie Salicylsäurederivate, vorzugsweise 4-lsopropylbenzylsalicylat, Salicylsäure-2-ethylhexylester, 3,3,5-Trimethyl-cyclohexylsalicylat, sowie 3-lmidazol-4-yl-acrylsäure und ihre Ester, 2-Phenylenbenzimidazol-5-sulfonsäure, Methylen bis-benzotriazolyltetramethylbutylphenol , 2-Cyan-3,3-diphenylacrylsäure, Butylmethoxy-dibenzoyl-methan, sowie Benzophenone oder Benzophenon-Derivate, vorzugsweise Benzophenon-3, Benzophenon-4, umfasst.

Als besonders geeignet im Sinne der vorliegenden Erfindung haben sich nachfolgende, im UV-Bereich absorbierende, Substanzen herausgestellt: 4- Bis(polyethoxyl)aminobenzoesäurepolyethoxyethylester, 2,2'-Dihydroxy-4,4'-dimethoxybenzophenon-5,5'di-Natriumsulfonat, 4-Methoxyzimtsäure-2-ethylhexylester, 3-(4'-Trimethylammonium)benzylidenbornan-2-on-Methylsulfat, 2,4,6-Trianilin-p-(carbo-2'-ethyl-hexyl- 1'-oxy)-1,3,5-triazin, Dioctyl Butamido Triazone, Bis-Ethylhexyloxyphnol Methoxyphenyltriazone, Terephthaloyliden-dicampher-sulfonsäure, Polymer von N-[2(und4)-(2-oxoborn-3-ylidenmethyl)benzyl]acrylamid, Drometriazol trisiloxan und 2,2'-(1,4-Phenylen)bis(1 H-benzimidazol-4,6-disulfonsäure, Mononatriumsalz)
Im Falle von Substanzen, die chemisch sauer reagieren wie Carbonsäuren oder Sulfonsäuren können auch deren pharmazeutisch akzeptable Salze wie vorzugsweise und ohne Anspruch auf Vollständigkeit K-, Na- und Triethanolamin(=TEA)-Salze zum Einsatz kommen.

Ferner kann das erfindungsgemäße halbfeste TTS als pharmazeutischenwirkstoff mindestens ein Hormon enthalten.

Weiterhin kann/können beim erfindungsgemäßen halbfesten TTS der/die pharmazeutische/n Wirkstoff/e Gestagen/e, vorzugsweise Gestoden oder Levonorgestrel sein.

Ferner kann erfindungsgemäß das halbfeste TTS System farblos und transparent sein.

Überraschenderweise konnte bei der Realisierung des erfindungsgemäßen halbfesten TTS aufgezeigt werden, dass die eingesetzten UV-Absorber ein geringeres Eindring- bzw. Durchdringvermögen bezogen auf die menschliche Haut, aufgezeigt im Vergleich zum zu schützenden pharmazeutischen Wirkstoff, besitzen. Es konnten somit halbfeste TTS ermittelt werden, bei welchen sich während des Anwendungszeitraumes der Lichtschutzstoff im Strahlengang des einfallenden Lichtes räumlich vor dem zu schützenden Wirkstoff anreichert, wodurch die Schutzwirkung noch erhöht wird.

Die Gelbildner der halbfesten TTS werden bevorzugt aus der Gruppe ausgewählt, die Cellulosen, vorzugsweise Hydroxyethylcellulose, Hydroxypropylcellulose, Methylcellulose, Methylhydroxyethylcellulose, Methylhydroxypropylcellulose, Na-Carboxylmethylcellulose, Chitosan-EDTA, Hochdisperses Siliziumdioxid, Gefälltes Siliziumdioxid, Bentonit, Stärken vorzugsweise, Maisstärke, Reisstärke, Kartoffelstärke, Weizenstärke, Carboxymethylamylopektin-Na, Tragant, Alginate, Polyacrylate, Polymethacrylate, Polyacrylat-Polyalkylacrylat-Crosspolymer, Acryloyldimethyltaurat/Vinylpyrrolidon Copolymer Pollyvinylalkohol, Polyvinylpyrrolidon, umfasst.

Die Erfindung wird durch nachfolgende Beispiele näher erläutert.

### Beispiel 1

Ketoprofen ist ein nichtsteroidales Antiphlogistikum (NSAID), das in der Form von Gels zur Behandlung von Schwellungen und Entzündungen der gelenknahen Weichteile (z. B. Sehnen, Sehnenscheiden, Bänder und Gelenkkapseln), insbesondere im Bereich der Schulter und des Ellenbogens; Sport- und Unfallverletzungen wie Prellungen, Verstauchungen, Zerrungen zum Einsatz kommt. Unter Einfluß von Sonnenlicht ist das Auftreten von phototoxischen und photoallergischen Hautreaktionen für derartige Gele beschrieben. Der Wirkstoff Ketoprofen wird durch den Zusatz des UV-Absorbers Uvinul DS 49 vor der lichtinduzierten Zersetzung geschützt.

| | |
|---|---|
| Ketoprofen | 2,5 % |
| Carbopol 940 | 1 % |
| Uvinul DS 49 | 2 % |
| (2,2'-Dihydroxy-4,4'-dimethoxybenzophenon-5,5'-di-Natriumsulfonat) | |
| Triethanolamin (TEA) | 1 % |
| Isopropanol | 20% |
| Propylenglycol | 20% |
| EDTA | 0,1% |
| Wasser ad | 100% |

Uvinul DS 49 hat ein Molekulargewicht von ca. 478,4 g/mol sowie einen rechnerischen LogP-Wert von -1.9.

Die Gelgrundlage wird aus Carbopol, TEA und Wasser nach den allgemein bekannten pharmazeutischen Vorschriften hergestellt. Danach wird Uvinul DS 49 und EDTA in diese Grundlage eingearbeitet. Isopropanol, Propylenglycol und Ketoprofen werden vermengt und anschließend in die Gelgrundlage eingearbeitet.

### Beispiel 2

Gestodenhaltige-Präparate können zur Hormonersatztherapie verwendet werden. Unter UV-Licht kann es zu einer Zersetzung des Wirkstoffes und damit einhergehend zu einem Wirkverlust kommen.

| | |
|---|---|
| Gestoden | 1 % |
| Uvinul MC 80 | 3% |
| (4-Methoxyzimtsäure 2-ethylhexyl ester) | |
| Diethylenglycolmonoethylether | 10% |
| Isopropylmyristat | 10% |
| Ethanol | 70% |
| Hydroxypropylcellose | 1,5 |
| Wasser ad | 100% |

Uvinul MC 80 hat ein Molekulargewicht von ca. 290 g/mol sowie einen rechnerischen LogP-Wert von 5.37.

Gestoden und Uvinul MC 80 werden in Ethanol gelöst. Anschließend werden Diethylenglycolmonoethylether, Isopropylmyristat und Wasser zugegeben und gut vermengt. Danach wird durch portionsweise Einarbeitung von Hydroypropylcellulose das Gel geformt und entsprechend der pharmazeutischen Vorschriften ausreichend quellen gelassen.

## Patentansprüche

1. Halbfestes transdermales therapeutisches System
mit einem einen lichtempfindlichen pharmazeutischen Wirkstoff
**dadurch gekennzeichnet, dass**
mindestens ein UV-Absorber ohne pharmakologische Wirksamkeit in den eingesetzten Konzentrationen enthalten ist und dieser im halbfesten TTS gelöst oder dispergiert vorliegt.

2. Halbfestes transdermales therapeutisches System
nach Anspruch 1, **dadurch gekennzeichnet,**
**dass** das System ein Gel ist.

3. Halbfestes transdermales therapeutisches System
nach Anspruch 1 oder 2, **dadurch gekennzeichnet,**
**dass** der/die Verteilungskoeffizient/en [log P] des/der UV-Absorber/s) entweder größer als 2,0 oder kleiner als 1,0 ist/sind, vorzugsweise größer als 3,0 oder kleiner als 0,0.

4. Halbfestes transdermales therapeutisches System
nach mindestens einem der vorhergehenden Ansprüche,
**dadurch gekennzeichnet, dass** die molare Masse des/der UV-Absorber/s größer als 150 gmol⁻¹ ist/sind, vorzugsweise größer als 250 gmol⁻¹.

5. Halbfestes transdermales therapeutisches System
nach einem oder mehreren der vorhergehenden Ansprüche,
**dadurch gekennzeichnet, dass** der/ die UV-Absorber in einem Anteil von 1-10% enthalten ist , vorzugsweise 2-5%, bezogen auf die Summe der Masse der nicht-leichtflüchtigen Vehikelbestandteile.

6. Halbfestes transdermales therapeutisches System
nach mindestens einem der vorhergehenden Ansprüche,
**dadurch gekennzeichnet, dass** die im UV-Bereich absorbierende/n Substanz/en aus der Gruppe ausgewählt ist/sind, die p-Aminobenzoesäure und Aminobenzoesäurederivate, vorzugsweise 4-Dimethylaminobenzoesäure-2-ethyl-hexylester, 4- bis(polyethoxyl)aminobenzoesäure-polyethoxyethylester, sowie Zimtsäure und ihre Derivate, vorzugsweise 4-Methoxyzimtsäureisoamylester, 4- Methoxyzimtsäure-2-ethylhexylester, sowie 3-Benzylidenbornan-2-on und Benzylidenbornan- 2-on-Derivate, vorzugsweise 3-(4')Methylbenzyliden-bornan- 2-on, 3-(4-Sulfo)benzylidenbornan-2-on, 3-(4'-Trimethylammonium)-benzylidenbornan-2-on-Methylsulfat, Polymer von N-[2(und4)-(2-oxoborn-3-ylidenmethyl)benzyl]acrylamid, sowie Salicylsäurederivate, vorzugsweise 4-Isopropylbenzylsalicylat, Salicylsäure-2-ethylhexylester, 3,3,5-Trimethyl-cyclohexylsalicylat, sowie 2,4,6-Trianilin-p-(carbo-2'-ethyl-hexyl- 1'-oxy)-1,3,5-triazin, Dioctyl Butamido Triazone, Bis-Ethylhexyloxyphnol Methoxyphenyltriazone, 3-Imidazol-4-yl-acrylsäure und ihre Ester, 2-Phenylenbenzimidazol-5-sulfonsäure und ihre K-, Na- und Triethanolamin(=TEA)-Salze, 2,2'-(1 ,4-Phenylen)bis(1Hbenzimidazol-4,6-disulfonsäure, Mononatriumsalz), Methylen bisbenzotriazolyl-tetramethylbutylphenol, Drometriazol trisiloxan, 2-Cyan-3,3-diphenylacrylsäure, Terephthaloyliden-dicampher-sulfonsäure, Butylmethoxy-dibenzoyl-methan, sowie Benzophenone oder Benzophenon-Derivate, vorzugsweise Benzophenon-3, Benzophenon-4, 2,2'-Dihydroxy-4,4'dimethoxybenzophenon-5,5'-di-Natriumsulfonat umfasst.

7. Halbfestes transdermales therapeutisches System
nach mindestens einem der vorhergehenden Ansprüche,
**dadurch gekennzeichnet, dass** als lichtempfindlicher pharmazeutischer Wirkstoff mindestens ein Hormon wirksam wird.

8. Halbfestes transdermales therapeutisches System
nach mindestens einem der vorhergehenden Ansprüche,
**dadurch gekennzeichnet, dass** der pharmazeutische Wirkstoff Gestagen, vorzugsweise Gestoden oder Levonorgestrel ist.

9. Halbfestes transdermales therapeutisches System
nach mindestens einem der vorhergehenden Ansprüche ,
**dadurch gekennzeichnet, dass** das System farblos und transparent ist.
